# EUROPÄISCHE PATENTSCHRIFT

(11) **EP 2 221 006 B1**
(45) Veröffentlichungstag und Bekanntmachung des Hinweises auf die Patenterteilung: **19.12.2012**
(21) Anmeldenummer: 10001135.2
(22) Anmeldetag: 04.02.2010
(51) Int. Cl.: A61B 17/04

(54) **Medizinisches Instrument zum Ergreifen von chirurgischem Nahtmaterial**
Medical instrument for gripping surgical sewing material
Instrument médical destiné à la préhension de matériau de suture chirurgical

(30) Priorität: 24.02.2009 DE 102009010101
(43) Veröffentlichungstag der Anmeldung: 25.08.2010
(73) Patentinhaber: Karl Storz GmbH & Co. KG, 78532 Tuttlingen (DE)
(72) Erfinder: Gerber, Christian, Prof., 8126 Zumikon (CH); Oberländer, Martin, 78234 Engen (DE); Berberich, Sascha, 78532 Tuttlingen (DE)
(74) Vertreter: Hofmeister, Frank

(56) Entgegenhaltungen:
- DE-C- 177 060
- DE-C1- 4 218 191
- US-A- 1 704 992
- US-A- 5 261 917
- US-A- 5 817 111
- US-A- 5 951 587
- US-B1- 6 183 484

## Beschreibung

Die Erfindung betrifft ein medizinisches Instrument zum Ergreifen von chirurgischem Nahtmaterial mit einem Schaft, an dessen proximalem Ende eine aus mindestens zwei Griffteilen bestehende Handhabe angeordnet ist und an dessen distalem Ende ein aus einem starren Maulteil und einem verschwenkbaren Maulteil bestehendes Werkzeug angeordnet ist, wobei das verschwenkbare Maulteil zum Öffnen und Schließen über ein verstellbar ausgebildetes Griffteil der Handhabe gegenüber dem starren Maulteil verschwenkbar ist, wozu das verschwenkbare Maulteil und das verstellbare Griffteil über ein im Schaft gelagertes Betätigungselement miteinander in Wirkverbindung stehen.

Derartige medizinische Fadenfassinstrumente kommen in der endoskopischen Chirurgie zum Einsatz, um durch Ergreifen des Nahtmaterials den Chirurgen in die Lage zu versetzen, im Körperinneren Nähte auszubilden.

Ein gattungsgemäßer Nadelhalter ist aus der US 5 951 587 A bekannt. Dieser bekannte Nadelhalter weist einen Schaft auf, an dessen distalem Ende ein aus einem starren Maulteil und einem verschwenkbaren Maulteil bestehendes Werkzeug angeordnet ist. Im starren Maulteil ist eine zum verschwenkbaren Maulteil hin sowie nach distal offene Vertiefung ausgebildet, die in der geschlossenen Position des Werkzeugs nur im Mittelbereich durch das verschwenkbare Maulteil abgedeckt wird. Aufgrund der distalseitigen Öffnung der Vertiefung ist ein sicheres Führen des ergriffenen Nahtmaterials nicht möglich, da immer die Gefahr besteht, dass das Nahtmaterial über das offene distale Ende entweicht.

Weiterhin ist aus der US 6 183 484 A eine Klemmvorrichtung für Nahtmaterial bekannt, die zwei verschwenkbare Griffteile mit zugehörigen Schäften aufweist, an deren Enden zwei Maulteile ausgebildet sind. Das starre Maulteil weist eine u-förmige, nach distal weisende Öffnung auf, die über einen Bügel des Maulteils so verschließbar ist, dass ein in der Öffnung angeordneter Faden nicht mehr nach distal aus der Öffnung entweichen kann. Bei geschlossenen Maulteilen verbleibt jedoch eine quer durch die Maulteile verlaufende Aussparung, die kein führendes Halten des Nahtmaterials ermöglicht.

Aus der US 5 817 111 A ist eine Greifvorrichtung für Nahtmaterial bekannt, die einen Schaft aufweist, in dem axialverschiebbar ein Rohr sowie ein Draht gelagert sind. Das distale Ende des Rohres ist aufgeschnitten rinnenförmig ausgebildet, wohingegen das distale Ende des Drahtes so nach unten abgewinkelt ausgebildet ist, dass der Draht, wenn er auf dem rinnenförmigen distalen Ende des Rohres aufliegt, mit dem Rohr eine seitlich offene Öse bildet, durch die ein Faden einführbar ist. Bei geschlossenen Maulteilen verbleibt jedoch diese seitlich offene Öse, die kein führendes Halten des Nahtmaterials ermöglicht.

Ein weiteres medizinisches Instrument ist beispielsweise aus der DE 198 48 958 A1 bekannt. Bei diesem bekannten Fadenfassinstrument ist im starren Maulteil des Werkzeugs eine quer durch das Maulteil verlaufende Aussparung ausgebildet, die zur Aufnahme des zu ergreifenden Nahtmaterials bei geschlossenem Werkzeug dient. Zwar lässt sich mit diesem bekannten Instrument das Nahtmaterial gut einfangen, jedoch ist die im starren Maulteil ausgebildete Aussparung zu groß, um das Nahtmaterial punktgenau der auszubildenden Nahtstelle zuführen zu können.

Davon ausgehend liegt der Erfindung die **Aufgabe** zugrunde, ein medizinisches Instrument zum Ergreifen von chirurgischem Nahtmaterial zu schaffen, das bei einfacher Handhabung ein sicheres Ergreifen und Führen des Nahtmaterials ermöglicht.

Die **Lösung** dieser Aufgabenstellung ist erfindungsgemäß dadurch gekennzeichnet, dass im starren Maulteil eine ausschließlich zum verschwenkbaren Maulteil hin offene, allseitig umrandete Mulde ausgebildet ist, die in der geschlossen Position des Werkzeugs durch das verschwenkbare Maulteil nur im Mittelbereich abdeckbar ist, wobei das verschwenkbare Maulteil mittig tailliert oder als schmaler Steg ausgebildet ist, so dass die Vertiefung in der geschlossenen Position des Werkzeugs zu beiden Seiten des verschwenkbaren Maulteils frei zugänglich ist und, dass das verschwenkbare Maulteil das ergriffene Nahtmaterial in der geschlossenen Position des Werkzeugs in die Vertiefung im starren Maulteil hineindrückt.

Durch die erfindungsgemäße Ausbildung der nur teilweise abdeckbaren Vertiefung im starren Maulteil wird bewirkt, dass das eingefangene Nahtmaterial bei geschlossenem Werkzeug U-förmig um das verschwenkbare Maulteil geführt in der Vertiefung des starren Maulteils angeordnet ist. Die U-förmige Umlenkung des Nahtmaterials ermöglicht ein Durchgleiten des Nahtmaterials durch die Maulteile bei gleichzeitig ausreichendem Halt, um das Nahtmaterial gezielt positionieren zu können.

Durch die schmalere Ausbildung des verschwenkbaren Maulteils in Relation zum starren Maulteil und insbesondere zur Breite der im starren Maulteil ausgebildeten Vertiefung wird die vorteilhafte U-förmige Umlenkung des ergriffenen Nahtmaterials um das verschwenkbare Maulteil und durch die Vertiefung erzielt.

Damit zusätzlich zum Ergreifen des Nahtmaterials auch ein festes Erfassen und möglicherweise Ziehen am Nahtmaterial möglich ist, wird mit der Erfindung weiterhin vorgeschlagen, dass am starren Maulteil zusätzlich zur Vertiefung eine ebene Fläche ausgebildet ist, an der das verschwenkbare Maulteil in der geschlossenen Position des Werkzeugs zumindest abschnittweise formschlüssig anliegt. Durch diese erfindungsgemäße Ausbildung der zusätzlichen ebenen Klemmfläche lässt sich das starre Maulteil in zwei Arbeitsbereiche aufteilen, nämlich den mit der Vertiefung versehenen Bereich zum Fadengleiten und den mit der ebenen Klemmfläche versehenen Bereich zum Fadenfassen. Vorzugsweise ist die ebene Fläche proximal von der Vertiefung am starren Maulteil ausgebildet ist.

Gemäß einer ersten praktischen Ausführungsform der Erfindung wird vorgeschlagen, dass sich das verschwenkbare Maulteil des Werkzeugs nach distal öffnet, so dass des erfindungsgemäße Instrument mit geöffnetem Maulteil voran auf das zu ergreifenden Nahtmaterial zugeführt werden kann.

Gemäß einer alternativen zweiten Ausführungsform der Erfindung wird vorgeschlagen, dass sich das verschwenkbare Maulteil des Werkzeugs retrograd, also mit der Öffnung zum proximalen Ende des Instruments gerichtet, öffnet.

Weiterhin wird mit der Erfindung vorgeschlagen, dass das distale Ende des starren Maulteils als scharfe Schneidspitze ausgebildet ist, um das zu nähende Gewebe mit dem ergriffenen Nahtmaterial im geschlossenen Werkzeug durchstoßen zu können.

Schließlich wird mit der Erfindung vorgeschlagen, dass am distalen Ende des verschwenkbaren Maulteils ein zum starren Maulteil hin weisender Fanghaken ausgebildet ist, um das Ergreifen und Halten des mit dem Werkzeug zu fassenden chirurgischen Nahtmaterials zu erleichtern.

Weitere Merkmale und Vorteile der Erfindung ergeben sich anhand der zugehörigen Zeichnungen, in denen ein Ausführungsbeispiel eines erfindungsgemäßen medizinischen Instruments zum Ergreifen von chirurgischem Nahtmaterial nur beispielhaft dargestellt ist, ohne die Erfindung auf dieses Ausführungsbeispiel zu beschränken. In den Zeichnungen zeigt:
- Fig. 1: eine schematische Seitenansicht eines erfindungsgemäßen medizinischen Instruments zum Ergreifen von chirurgischem Nahtmaterial;
- Fig. 2: eine vergrößerte perspektivische Ansicht des Details II gemäß Fig. 1, das Werkzeug in der geöffneten Position darstellend;
- Fig. 3: eine perspektivische Ansicht gemäß Fig. 2, jedoch das Werkzeug in der geschlossenen Position darstellend;
- Fig. 4: eine teilweise geschnittene Seitenansicht der Darstellung gemäß Fig. 2;
- Fig. 5: eine teilweise geschnittene Seitenansicht der Darstellung gemäß Fig. 3, jedoch mit ergriffenem Nahtmaterial;
- Fig. 6: eine Draufsicht auf die Darstellung gemäß Fig. 3, jedoch mit ergriffenem Nahtmaterial und
- Fig. 7: einen Schnitt entlang der Linie VII-VII gemäß Fig. 6.

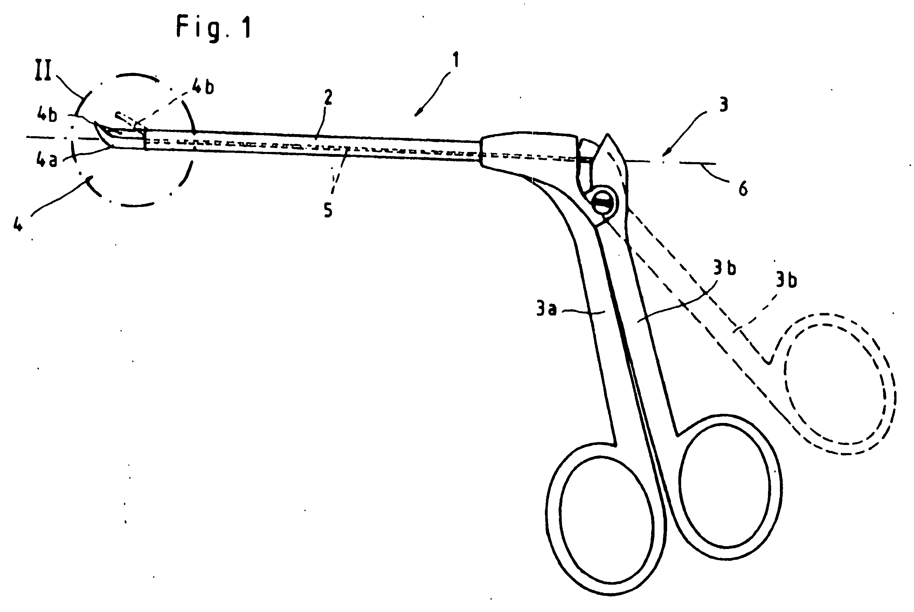

Die Abbildung Fig. 1 zeigt ein als zum Ergreifen von chirurgischem Nahtmaterial ausgebildetes medizinisches Instrument.

Das nur schematisch dargestellte medizinische Instrument 1 besteht im Wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, das aus einem starren Maulteil 4a und einem gegenüber dem starren Maulteil 4a verschwenkbaren Maulteil 4b besteht. Welches der beiden Griffteile 3a und 3b der Handhabe 3 verschwenkbar oder anderweitig verstellbar ausgebildet ist, ist für die Funktionsweise des medizinischen Instruments 1 nicht relevant.

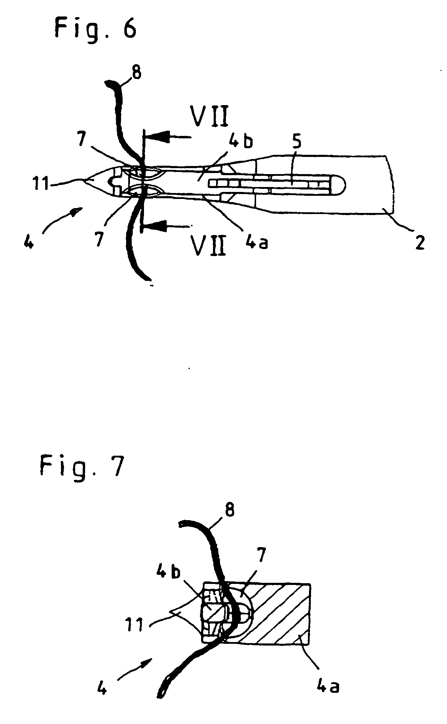

Wie aus Fig. 1 weiterhin ersichtlich, stehen das verschwenkbare Maulteil 4b des Werkzeugs 4 und der verschwenkbare Griffteil 3b der Handhabe 3 über ein in dem hohlen Schaft 2 axial verschiebbar gelagertes Betätigungselement 5 derart in Wirkverbindung miteinander, dass das Verstellen des Griffteils 3b der Handhabe 3 das verschwenkbare Maulteil 4b des Werkzeugs 4 von der geschlossenen Stellung (durchgezogene Darstellung gemäß Fig. 1 sowie Fig. 3 und Fig. 5 bis 7) in die geöffnete Stellung (gestrichelte Darstellung gemäß Fig. 1 sowie Fig. 2 und 4) bzw. umgekehrt überführbar ist. Die jeweils zugehörige Stellung des verschwenkbaren Griffteils 3b der Handhabe 3 ist in der Abbildung Fig. 1 ebenfalls durchgezogen (für die geschlossene Stellung) und gestrichelt (für die geöffnete Stellung) dargestellt.

Bei der dargestellten Ausführungsform ist das Werkzeug 4 so ausgebildet, dass die Maulteile 4a und 4b im Wesentlichen in Verlängerung zur Instrumentenlängsachse 6 ausgebildet sind. Selbstverständlich ist es auch möglich das Werkzeug 4 so auszubilden, dass die Maulteile 4a und 4b zur Instrumentenlängsachse 6 abgewinkelt sind. Ebenso ist es im Gegensatz zu der dargestellten geraden Ausbildung des Schaftes 2 möglich, das distale Ende des Schaftes 2 gebogen auszubilden.

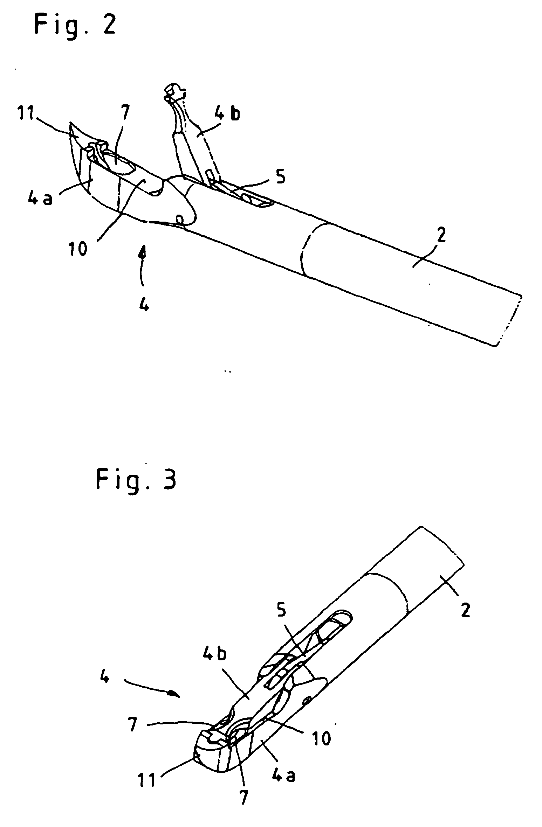

Wie weiterhin insbesondere aus den Abbildungen Fig. 2, 4 und 5 ersichtlich, ist das verschwenkbare Maulteil 4b des Werkzeugs 4 so am Betätigungselement 5 gelagert, dass sich das proximal angelenkte verschwenkbare Maulteil 4b nach distal öffnet. Alternativ ist es selbstverständlich auch möglich, das Werkzeug 4 so auszubilden, dass sich das verschwenkbare Maulteil 4b distal seitig angelenkt retrograd, also mit der Öffnung zum proximalen Ende des medizinischen Instruments 1 gerichtet, öffnet.

Der Aufbau der Maulteile 4a und 4b des Werkzeugs 4 ergibt sich aus den Abbildungen Fig. 2 bis 7.

Wie insbesondere aus Fig. 2 und 3 ersichtlich, ist im starren Maulteil 4a des Werkzeugs 4 eine nur zum verschwenkbaren Maulteil 4b hin offene Vertiefung 7 ausgebildet, die im in der geschlossenen Position des Werkzeugs 4 (Fig. 3) durch das verschwenkbare Maulteil 4b nur im Mittelbereich abgedeckt wird, so dass die Vertiefung 7 auch bei geschlossenem Werkzeug 4 zu beiden Seiten des verschwenkbaren Maulteils 4b frei zugänglich bleibt.

Bei der dargestellten Ausführungsform wird diese nur teilweise Abdeckung der Vertiefung 7 durch das verschwenkbare Maulteil 4b dadurch erzielt, dass das verschwenkbare Maulteil 4b mittig tailliert, also mit einer im Mittelbereich reduzierten Breite, ausgebildet ist. Alternativ ist es beispielsweise auch möglich, das verschwenkbare Maulteil 4b insgesamt als schmalen Steg auszubilden, der die Vertiefung 7 im starren Maulteil 4a nur im Mittelbereich abdecken kann.

Um das Ergreifen und Halten eines mit dem Werkzeug 4 zu fassenden chirurgischen Nahtmaterials 8 zu erleichtern, ist am distalen Ende des verschwenkbaren Maulteils 4b ein zum starren Maulteil 4a hin weisender Fanghaken 9 ausgebildet.

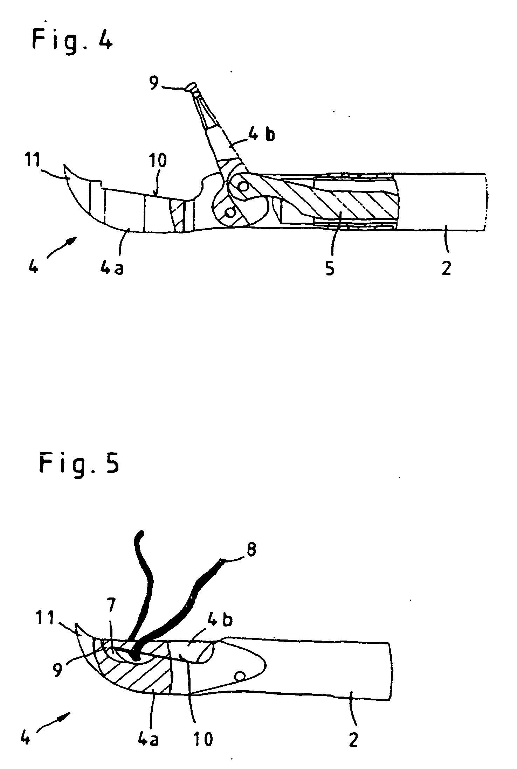

Das Ausbilden der nur zum verschwenkbaren Maulteil 4b hin offenen Vertiefung 7 im starren Maulteil 4a führt dazu, dass das ergriffene Nahtmaterial 8, wie in den Abbildungen Fig. 5, 6 und 7 dargestellt, in der geschlossenen Position des Werkzeugs 4 durch das verschwenkbare Maulteil 4b in die Vertiefung 7 im starren Maulteil 4a hineingedrückt wird und U-förmig um das verschwenkbare Maulteil 4b geführt in der Vertiefung 7 des starren Maulteils 4a angeordnet ist.

Diese U-förmige Umlenkung des Nahtmaterials 8 ermöglicht zwar weiterhin ein Durchgleiten des Nahtmaterials 8 durch die Maulteile 4a, 4b, jedoch bei gleichzeitig ausreichendem Halt, um das Nahtmaterial 8 gezielt positionieren zu können.

Bei der dargestellten Ausführungsform des medizinischen Instruments 1 kann das Halten und Führen des vom Werkzeug 4 ergriffenen Nahtmaterials 8 dadurch verbessert werden, dass am starren Maulteil 4a des Werkzeugs 4 zusätzlich zu der Vertiefung 7 eine ebene Fläche 10 ausgebildet ist, an der das verschwenkbare Maulteil 4b in der geschlossenen Position des Werkzeugs 4 das Nahtmaterial 8 klemmend ergreifend zumindest abschnittweise formschlüssig anliegt, wie dies der Abbildung gemäß Fig. 5 zu entnehmen ist.

Durch diese Ausbildung der zusätzlichen ebenen Klemmfläche 10 lässt sich das starre Maulteil 4a in zwei Arbeitsbereiche aufteilen, nämlich den mit der Vertiefung 7 versehenen Bereich zum Fadengleiten und den mit der ebenen Fläche 10 versehenen Bereich zum Fadenfassen. Vorzugsweise ist die ebene Fläche 10 proximal von der Vertiefung 7 am starren Maulteil 4a ausgebildet.

Wie weiterhin insbesondere aus den Abbildungen Fig. 2 bis 7 ersichtlich, ist das distale Ende des starren Maulteils 4a als scharfe Schneidspitze 11 ausgebildet ist, um das zu nähende Gewebe mit dem ergriffenen Nahtmaterial 8 im geschlossenen Werkzeug 4 durchstoßen zu können.

Ein solchermaßen ausgestaltetes medizinisches Instrument 1 zum Ergreifen von chirurgischem Nahtmaterial 8 zeichnet sich dadurch aus, dass durch die Ausbildung der Vertiefung 7 im starren Maulteil 4a das ergriffenen Nahtmaterial 8 U-förmig um das verschwenkbare Maulteil 4b geführt wird, wodurch ein sicheres Ergreifen und Führen des Nahtmaterials 8 ermöglicht wird.

### Bezugszeichenliste

- 1: medizinisches Instrument
- 2: Schaft
- 3: Handhabe
- 3a: starres Griffteil
- 3b: verschwenkbares Griffteill
- 4: Werkzeug
- 4a: starres Maulteil
- 4b: verschwenkbares Maulteil
- 5: Betätigungselement
- 6: Instrumentenlängsachse
- 7: Vertiefung
- 8: Nahtmaterial
- 9: Fanghaken
- 10: ebene Fläche
- 11: Schneidspitze

## Patentansprüche

1. Medizinisches Instrument zum Ergreifen von chirurgischem Nahtmaterial mit einem Schaft (2), an dessen proximalem Ende eine aus mindestens zwei Griffteilen (3a, 3b) bestehende Handhabe (3) angeordnet ist und an dessen distalem Ende ein aus einem starren Maulteil (4a) und einem verschwenkbaren Maulteil (4b) bestehendes Werkzeug (4) angeordnet ist, wobei das verschwenkbare Maulteil (4b) zum Öffnen und Schließen über ein verstellbar ausgebildetes Griffteil (3b) der Handhabe (3) gegenüber dem starren Maulteil (4a) verschwenkbar ist, wozu das verschwenkbare Maulteil (4b) und das verstellbare Griffteil (3b) über ein im Schaft (2) gelagertes Betätigungselement (5) miteinander in Wirkverbindung stehen.
**dadurch gekennzeichnet,**
**dass** im starren Maulteil (4a) eine Vertiefung (7) als ausschließlich zum verschwenkbaren Maulteil (4b) hin offene, allseitig umrandete Mulde ausgebildet ist, die in der geschlossen Position des Werkzeugs (4) durch das verschwenkbare Maulteil (4b) nur im Mittelbereich abdeckbar ist, wobei das verschwenkbare Maulteil (4b) mittig tailliert oder als schmaler Steg ausgebildet ist, so dass die Vertiefung (7) in der geschlossenen Position des Werkzeugs (4) zu beiden Seiten des verschwenkbaren Maulteils (4b) frei zugänglich ist und, dass das verschwenkbare Maulteil (4b) das ergriffene Nahtmaterial (8) in der geschlossenen Position des Werkzeugs (4) in die Vertiefung (7) im starren Maulteil (4a) hineindrückt.

2. Medizinisches Instrument nach der Anspruch 1, **dadurch gekennzeichnet, dass** am starren Maulteil (4a) zusätzlich zur Vertiefung (7) eine ebene Fläche (10) ausgebildet ist, an der das verschwenkbare Maulteil (4b) in der geschlossenen Position des Werkzeugs (4) zumindest abschnittweise formschlüssig anliegt.

3. Medizinisches Instrument nach Anspruch 2, **dadurch gekennzeichnet, dass** die ebene Fläche (10) proximal von der Vertiefung (7) am starren Maulteil (4a) ausgebildet ist.

4. Medizinisches Instrument nach einem der Ansprüche 1 bis 3, **dadurch gekennzeichnet, dass** sich das verschwenkbare Maulteil (4b) des Werkzeugs (4) nach distal öffnet.

5. Medizinisches Instrument nach einem der Ansprüche 1 bis 4, **dadurch gekennzeichnet, dass** sich das verschwenkbare Mauteil (4b) des Werkzeugs (4) retrograd öffnet.

6. Medizinisches Instrument nach einem der Ansprüche 1 bis 5, **dadurch gekennzeichnet, dass** das distale Ende des starren Maulteils (4a) als scharfe Schneidspitze (11) ausgebildet ist.

7. Medizinisches Instrument nach einem der Ansprüche 1 bis 6, **dadurch gekennzeichnet, dass** am distalen Ende des verschwenkbaren Maulteils (4b) ein zum starren Maulteil (4a) hin weisender Fanghaken (9) ausgebildet ist.

## Claims

1. A medical instrument for gripping surgical suture material, comprising a shaft (2), at the proximal end of which a handle (3) consisting of at least two grip members (3a, 3b) is arranged, and at the distal 1 end of which a tool (4) consisting of a rigid jaw member (4a) and a pivotable jaw member (4b) is arranged, wherein for opening and closing, the pivotable jaw member (4b) is pivotable with respect to the rigid jaw member (4a) via a displaceably configured grip member (3b) of the handle (3), for which purpose the pivotable jaw member (4b) and the displaceable grip member (3b) are in operative connection with each other via an actuation element (5) mounted in the shaft (2),
**characterized in**
**that** in the rigid jaw member (4a) an indentation (7) is formed as a trough which is bordered on all sides and is open exclusively toward the pivotable jaw member (4b), and which trough, in the closed position of the tool (4), can be covered by the pivotable jaw member (4b) only in the central area, wherein the pivotable jaw member (4b) is tapered in the middle or is formed as a narrow bar so that in the closed position of the tool (4), the indentation (7) is freely accessible on both sides of the pivotable jaw member (4b), and that in the closed position of the tool (4), the pivotable jaw member (4b) presses the gripped suture material (8) into the indentation (7) in the rigid jaw member (4a).

2. The medical instrument according to claim 1, **characterized in that** in addition to the indentation (7) on the rigid jaw member (4a), a plane surface (10) is formed against which, in the closed position of the tool (4), the pivotable jaw member (4b) rests at least in sections in a form-locking manner.

3. The medical instrument according to claim 2, **characterized in that** the plane surface (10) is formed proximally from the indentation (7) on the rigid jaw member (4a).

4. The medical instrument according to any one of the claims 1 to 3, **characterized in that** the pivotable jaw part (4b) of the tool (4) opens in the distal direction.

5. The medical instrument according to any one of the claims 1 to 4, **characterized in that** the pivotable jaw member (4b) of the tool opens in the retrograde direction.

6. The medical instrument according to any one of the claims 1 to 5, **characterized in that** the distal end of the rigid jaw member (4a) is formed as a sharp cutting point (11).

7. The medical instrument according to any one of the claims 1 to 6, **characterized in that** on the distal end of the pivotable jaw member (4b), a catch hook (9) is formed which points toward the rigid jaw member (4a).

## Revendications

1. Instrument médical pour la préhension d'un matériau de suture chirurgical, pourvu d'une tige (2), à l'extrémité proximale de laquelle est disposée une poignée (3) composée d'au moins deux pièces de poignée (3a, 3b) et à l'extrémité distale de laquelle est disposé un outil (4) constitué d'une pièce de mâchoire fixe (4a) et d'une pièce de mâchoire pivotante (4b), la pièce de mâchoire pivotante (4b) pouvant, au moyen d'une pièce (3b) de la poignée (3) réalisée de manière à être mobile, pivoter pour l'ouverture et la fermeture par rapport à la pièce de mâchoire fixe (4a), la pièce de mâchoire pivotante (4b) et la pièce de poignée mobile (3b) étant à cet effet en liaison de coopération entre elles au moyen d'un élément d'actionnement (5) logé dans la tige (2),
**caractérisé**
**en ce qu'**un évidement est ménagé dans la pièce de mâchoire fixe (4a) comme poche exclusivement ouverte vers la pièce de mâchoire pivotante (4b) et bordée de tous côtés, laquelle ne peut être recouverte que dans sa partie centrale par la pièce de mâchoire pivotante (4b) en position de fermeture de l'outil (4), la pièce de mâchoire pivotante (4b) étant cintrée en son milieu ou réalisée comme une entretoise étroite, si bien que l'évidement (7) est librement accessible de part et d' autre de la pièce de mâchoire pivotante (4b) en position de fermeture de l'outil (4) et que la pièce de mâchoire pivotante (4b) serre le matériau de suture (8) saisi dans l'évidement (7) de la pièce de mâchoire fixe (4a) en position de fermeture de l'outil (4).

2. Instrument médical selon la revendication 1, **caractérisé en ce qu'**une surface plane (10) est formée sur la pièce de mâchoire fixe (4a) en plus de l'évidement (7), contre laquelle la pièce de mâchoire pivotante (4b) repose avec une correspondance au moins partielle de forme en position de fermeture de l'outil (4).

3. Instrument médical selon la revendication 2, **caractérisé en ce que** la surface plane (10) est réalisée de manière à être proximale à l'évidement (7) sur la pièce de mâchoire fixe (4a).

4. Instrument médical selon l'une des revendications 1 à 3, **caractérisé en ce que** la pièce de mâchoire pivotante (4b) de l'outil (4) s'ouvre dans la direction distale.

5. Instrument médical selon l'une des revendications 1 à 4, **caractérisé en ce que** la pièce de mâchoire pivotante (4b) de l'outil (4) s'ouvre vers l'arrière.

6. Instrument médical selon l'une des revendications 1 à 5, **caractérisé en ce que** l'extrémité distale de la pièce de mâchoire fixe (4a) est réalisée comme dent (11) de coupe.

7. Instrument médical selon l'une des revendications 1 à 6, **caractérisé en ce qu'**un crochet de préhension (9) dirigé vers la pièce de mâchoire fixe (4a) est formé à l'extrémité distale de la pièce de mâchoire pivotante (4b).
